# EUROPEAN PATENT APPLICATION

(11) **EP 2 301 515 A1**
(43) Date of publication of application: **30.03.2011**
(21) Application number: 10009626.2
(22) Date of filing: 15.09.2010
(51) Int. Cl.: A61K 6/083

(54) **Dental restorative material composition**

(30) Priority: 25.09.2009 JP 2009220988
(71) Applicant: GC Corporation, Itabashi-ku Tokyo 174-8585 (JP)
(72) Inventor: Machida, Daiki, Tokyo 174-8585 (JP); Suzuki, Takumi, Tokyo 174-8585 (JP); Niwa, Tomoaki, Tokyo 174-8585 (JP)
(74) Representative: Müller-Boré & Partner Patentanwälte

(57) **Abstract**

To provide a dental restorative material composition having good surface lubrication and high strength without causing uneven abrasion, a dental restorative material composition includes 10 to 50% by weight of an organic-inorganic composite filler having an average particle diameter of 5 to 60µm, a (meth)acrylate compound, an inorganic filler having an average primary particle diameter of 0.1 to 0.4µm, and a photopolymerization initiator, where the organic-inorganic composition is produced by mixing a (meth)acrylate compound and 60 to 90% by weight of an inorganic filler having an average primary particle diameter of 0.1 to 0.4µm, curing the mixture, and pulverizing the cured material, and a difference between a ratio (% by weight) of the (meth)acrylate compound in the organic-inorganic composite filler and a ratio (% by weight) of the (meth)acrylate compound in the entire composition is within ±5 (% by weight).

## Description

The present invention relates to a dental restorative material composition used for restoring a defect of a tooth.

Conventionally, materials such as metals and an amalgam have been used for restoring a defect of a tooth due to a dental caries. However, in recent years, dental restorative materials such as a composite resin and a hard resin have been widely used, and those dental restorative materials can make a similar color tone to that of a natural tooth, compared with the metals. One of restorative methods is a direct restorative method including the steps of coating a dental bonding material in a cavity formed at a tooth, thereafter directly filling a dental restorative material into the cavity, and polymerizing and curing the dental restorative material in the cavity by chemical polymerization or photopolymerization. Another method is an indirect restorative method including the steps of collecting an intraoral impression of a cavity formed at a tooth, producing an intraoral gypsum model based on the impression, producing a dental restorative material on the intraoral gypsum model, and fixing the dental restorative material in the cavity by a dental bonding material.

The dental restorative material basically includes a polymerizable monomer, a filler, and a polymerization initiator, and has been improved many times to look for strength and aesthetic property. In many cases, characteristics of the dental restorative material are determined depending on a filling rate or an average particle diameter of filler powder. For example, a dental restorative material including, as a filler, glass powder having an average particle diameter of about 100µm has good mechanical strength. However, this dental restorative material has low polishing property because of having a too large particle diameter, so that a surface having glossiness cannot be obtained. In addition, the dental restorative material has further problems, in which a resin part of a surface on the cured material is abraded selectively in an oral cavity, and glass powder projects to have a file-like shape, so that the project part abrades a pairing human tooth or dental material.

For solving the above-mentioned problems, Japanese Patent Application Laid-Open No. 62-089701, Japanese Patent Publication No. 62-086003, and Japanese Patent Publication No. 1-057082 propose a dental restorative material including about 60 to 90% by weight of a spherical inorganic filler having a particle diameter within a range from 0.1µm to 1µm and a uniform particle diameter distribution. Since the inorganic filler has a spherical shape and a uniform particle diameter, such a dental restorative material can be blended with a polymerizable monomer with a high blending ratio, so that the material has good mechanical strength and surface glossiness. However, there is a problem that the spherical inorganic filler powder is easy to fall out from the resin.

Further, a dental restorative material including, as an inorganic filler, an ultrafine particle inorganic filler having a particle diameter equal to or smaller than 0.1µm has been also proposed. When this filler is used for the dental restorative material, the material can have good surface glossiness, and is hard to abrade a pairing human tooth or dental material. However, since the ultrafine particle inorganic filler has a very large specific surface area, the filler can only be blended in the dental restorative material about 20 to 30% by weight with the maximum. As a result, a ratio of a polymerizable monomer in the dental restorative material becomes high, and thereby the material has high polymerization shrinkage, and has low abrasion resistance and low strength. Therefore, the dental restorative material is poor in a physical property.

For solving the above-mentioned problems, Japanese Patent Application Laid-Open No. 54-107187 and Japanese Patent Application Laid-Open No. 56-020066 proposes a dental restorative material using a composite filler which is produced by coating inorganic filler powder having an average particle diameter of equal to or smaller than 0.1µm with a polymerizable monomer. Further, Japanese Patent Application Laid-Open No. 5-194135 discloses a dental restorative material using an organic-inorganic composite filler having an average particle diameter of 5 to 50µm, where the organic-inorganic composite filler is produced by mixing glass powder having an average particle diameter of 1 to 2µm and a monomer, polymerizing and curing the mixture, and thereafter pulverizing the cured material. In the organic-inorganic composite filler, a specific surface area is relatively suppressed to be low as a filler, although the composite filler includes inorganic fine particles having an average particle diameter of 5 to 50nm. Thus, the dental restorative material can include a large amount of the filler and suppress polymerization shrinkage, and further can have good surface lubrication. However, since a boundary bonding between a composition matrix and the organic-inorganic composite filler is weak, the entire composition cannot have high strength. Therefore, for strengthening bonding between the matrix of the dental restorative material composition and the organic-inorganic composite filler, a surface treatment of the organic-inorganic composite filler is performed by using an organosilicon compound. However, since the organosilicon compound affects on only inorganic matters, the effect of the organosilicon compound to the surface treatment of the organic-inorganic composite filler is low, because a composition ratio of organic components is high in the organic-inorganic composite filler. Thus, there are problems that, for example, an end part of the dental restorative material is chipped. Further, in the entire composition, the ratio of the inorganic components is low, so that abrasion resistance is low.

For improving strength and abrasion resistance of the dental restorative material, for example, Japanese Patent Application Laid-Open No. 2000-080013 discloses a dental restorative material including about 10 to 60% by weight of the aforementioned organic-inorganic composite filler and about 5 to 60% by weight of glass powder having an average particle diameter of 0.5 to 2µm. The dental restorative material is easy to abrade unevenly due to a difference in the characteristics between the organic-inorganic composite filler and the inorganic filler. The inorganic filler having an average particle diameter of about 2µm, which is used for improving strength, causes decreasing of surface lubrication. Furthermore, the problem that the organic-inorganic composite filler is difficult in performing of the surface treatment still remains unsolved, and the strength is not good.

The present invention is directed to a dental restorative material composition capable of having good surface lubrication and high strength without causing uneven abrasion.

Present inventors found out the followings to complete the present invention. A dental restorative material composition includes 10 to 50% by weight of an organic-inorganic composite filler having an average particle diameter of 5 to 60µm, a (meth)acrylate compound, an inorganic filler having an average primary particle diameter of 0.1 to 0.4µm, and a photopolymerization initiator. The organic-inorganic composite filler is produced by mixing a (meth)acrylate compound and 60 to 90% by weight of an inorganic filler having an average primary particle diameter of 0.1 to 0.4µm, curing the mixture, and pulverizing the cured material. In the dental restorative material composition, a difference between a ratio (% by weight) of the (meth)acrylate compound in the organic-inorganic composite filler and a ratio (% by weight) of the (meth)acrylate compound in the entire composition is within ±5 (% by weight). According to this dental restorative material composition, since the difference of the ratio of the inorganic filler in the composition is small between the inorganic-organic composite filler and the entire composition, the dental restorative material composition is hard to abrade unevenly. Furthermore, since the inorganic filler has a specific particle diameter, a blending ratio of the inorganic filler to the organic-inorganic composite filler can increase, and surface lubrication is also good.

A dental restorative material composition according to the present invention can have uneven abrasion resistance, good polishing property, surface lubrication, and strength.

An organic-inorganic composite filler used for a dental restorative material composition according to the present invention is produced by mixing a monomer of (meth)acrylate as mentioned below and an inorganic filler having an average primary particle diameter of 0.1 to 0.4µm so as to make a ratio of the inorganic filler to be 60 to 90% by weight, polymerizing and curing the mixture, and pulverizing the cured material to have an average particle diameter of 5 to 60µm. As a curing agent at a time of polymerization, a conventional polymerization catalyst can be used. An organic peroxide and an azo compound can be used when heat-curing. A photopolymerization initiator can be used when photo-curing is performed. In addition, chemical polymerization or the like at an ordinary temperature can be also used.

The organic-inorganic composite filler has an average particle diameter of 5 to 60µm. When the average particle diameter is smaller than 5µm, a specific surface area is too large, and a blendable amount with respect to the dental restorative material composition comes to be low. Thus, the strength of the dental restorative material composition after polymerizing and curing is reduced. When the average particle diameter exceeds 60µm, the strength of the dental restorative material composition is reduced. It is preferable that a blending ratio of the organic-inorganic composite filler to the dental restorative material composition is 10 to 50% by weight. When the blending ratio is less than 10% by weight, operability of a paste-like dental restorative material composition before polymerization is reduced. When the blending ratio exceeds 50% by weight, the strength of the dental restorative material composition after polymerization is reduced. More preferably, the blending ratio is 25 to 50% by weight.

The (meth)acrylate compound used for the dental restorative material composition according to the present invention is a monomer, an oligomer, or a prepolymer of acrylate or methacrylate, and publicly known materials used for a dental material can be used. More particularly, the (meth)acrylate compound used in the present invention could be methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfryl (meth)acrylate, glycidyl (meth)acrylate, 2-hydroxyethyl (meth)acrylate, 2-hydroxypropyl (meth)acrylate, 3-hydroxypropyl (meth)acrylate, 2-ethoxyethyl (meth)acrylate, 2-methoxyethyl (meth)acrylate, 2-ethylhexyl

(meth)acrylate, benzyl (meth)acrylate, 2-hydroxy-1, 3-di(meth)acryloxy propane, ethylene glycol di (meth) acrylate, diethylene glycol di (meth) acrylate, triethylene glycol di(meth)acrylate, butylene glycol di(meth)acrylate, neopentyl glycol di(meth)acrylate, 1, 3-butanediol di(meth)acrylate, 1, 4-butanediol di(meth)acrylate, 1, 6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di (meth) acrylate, bisphenol A polyethoxy methacrylate, bisphenol A diglycidyl (meth)acrylate or the like. Further, as for (meth)acrylate having urethane bond, di-2-(meth)acryloxyethyl-2, 2, 4-trimethylhexamethylene dicarbamate, 1, 3, 5-tris [1, 3-bis{(meth)acryloyloxy}-2-propoxycarbonylaminohex ane]-1, 3, 5-(1H, 3H, 5H) triazine-2, 4, 6- trione. In addition, the (meth)acrylate having urethane bond could be (meth) acrylate of urethane oligomer including 2, 2'-di(4-hydroxycyclohexyl) propane, 2-oxypanone, hexamethylene diisocyanate, and 2-hydroxyethyl (meth)acrylate, and (meth)acrylate of urethane oligomer including 1, 3-butanediol, hexamethylene diisocyanate, and 2-hydroxyethyl (meth)acrylate, and the like. It is preferable that a blending ratio of the (meth)acrylate compound to the entire dental restorative material composition is 10 to 30% by weight. When the blending ratio is less than 10% by weight, the paste of the dental restorative material composition before polymerizing is hard and operability reduces. When the blending ratio exceeds 30% by weight, the abrasion resistance and strength of the dental restorative material composition after polymerization are reduced. More preferably, the blending ratio is 15 to 25% by weight.

The inorganic filler having an average primary particle diameter of 0.1 to 0.4µm is glass powder mainly including SiO₂, SrO, B₂O₃, BaO, Al₂O₃, Na₂O, CaO, ZnO, La₂O₃, WO₃, ZrO₂, TiO₂, Nb₂O₅, F, or the like. It is preferable that a blending ratio of the inorganic filler to the entire dental restorative material composition is 70 to 90% by weight. When the blending ratio is less than 70% by weight, the strength of the dental restorative material composition after polymerization is reduced. When the blending ratio exceeds 90% by weight, the paste of the dental restorative material composition before polymerization becomes hard, and the operability tends to decrease. More preferably, the blending ratio is 75 to 85% by weight.

The organic-inorganic composite filler used for the dental restorative material composition according to the present invention is a powder component produced by mixing a (meth)acrylate compound and an inorganic filler having an average primary particle diameter of 0.1 to 0.4µm so as to have a ratio of the inorganic filler of 60 to 90% by weight, curing the mixture, and pulverizing the cured material so as to have an average particle diameter of 5 to 60µm. It is necessary that a difference between a ratio (% by weight) of the (meth)acrylate compound in the organic-inorganic composite filler and a ratio (% by weight) of the (meth)acrylate compound in the entire dental restorative material composition is within ±5 (% by weight). When the difference is not within ±5 (% by weight), the dental restorative material composition after polymerization causes uneven abrasion.

The photopolymerization initiator that is combination of a sensitizing material and a reducing material is used in general. The sensitizing material could be camphorquinone, benzyl, diacetyl, benzyl dimethyl ketal, benzyl diethyl ketal, benzyl di(2-methoxyethyl) ketal, 4,4'-dimethylbenzyl-dimethyl ketal, anthraquinone, 1-chloroanthraquinone, 2-chloroarithraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluorothioxanthone, thioxanthone-10,10-dioxide, thioxanthone-10-oxide, benzoin methyl ether, benzoin ethyl ether, isopropyl ether, benzoin isobutyl ether, benzophenone, bis(4-dimethylaminophenyl) ketone, 4,4'-bisdiethylaminobenzophenone, a compound including an azide group, and the like. These sensitizing materials can be used independently or by mixing one or more kinds.

Tert-amine is used as a reducing material in general. Preferably the tert-amine could be dimethylaminoethyl methacrylate, triethanolamine, methyl 4-dimethylaminobenzoate, ethyl 4-dimethylaminobenzoate, or isoamyl 4-dimethylaminobenzoate. In addition to these reducing materials, benzoyl peroxide, an organic metal compound, a sulfinic acid derivative, and the like, can be used as a reducing material. In such a photopolymerization type dental restorative material composition, a polymerization reaction can be attained by irradiating active rays such as ultraviolet rays, visible rays, or the like.

As a light source, various kinds of ultrahigh-pressure, high-pressure, medium-pressure or low-pressure mercury lamps, a chemical lamp, a carbon arc lamp, a metal halide lamp, a fluorescent lamp, a tungsten lamp, a xenon lamp, an argon ion laser, and the like can be used. In addition to those, the dental restorative material composition according to the present invention can include minute amounts of an ultraviolet absorbent, a coloring agent, a polymerization inhibitor, or the like according to necessity.

The dental restorative material composition according to the present invention can include glass fine powder having an average particle diameter of 0.01 to 0.1µm within a range from 0.1 to 5%.

### [Example]

Particular examples will be described below. <Producing method of an organic-inorganic composite filler>

A organic-inorganic composite filler (reference symbol A in Table 1) was produced by mixing a monomer of (meth)acrylate (reference symbol B in Table 1) and an inorganic filler (reference symbol C in Table 1) as glass powder mainly including SiO₂ at ratios shown in Table 1, heating and polymerizing the mixture at 130°C for 2 hours, and pulverizing the polymerized material by a ball mill to have a particle diameter shown in Table 1. In addition, 1% by weight of α,α'-azobisisobutyronitrile was used as a curing agent.

**Table 1 (% by weight)**

| | | A1 | A2 | A3 | A4 | A5 | A6 | Acomp.1 | Acomp.2 | Acomp.3 |
|---|---|---|---|---|---|---|---|---|---|---|
| B | UDMA | | 20 | | 29 | 28 | 40 | | 28 | |
| | Bis-GMA | 20 | | 10 | | | | 40 | | 27 |
| | NPGDMA | 8 | 9 | 9 | | | | 10 | | |
| Total of B components | | 28 | 29 | 19 | 29 | 28 | 40 | 50 | 28 | 27 |
| C | Particle Diameter 0.1µm | | 71 | | 21 | | | | | |
| | Particle Diameter 0.4µm | 72 | | 81 | 50 | 72 | 60 | 50 | 72 | |
| | Particle Diameter 2.0µm | | | | | | | | | 73 |
| Average particle diamter of A component (µm) | | 20 | 20 | 20 | 20 | 50 | | 20 | 2 | 20 |

| | | | | | | | | | | |
|---|---|---|---|---|---|---|---|---|---|---|
| UDMA: Di-2-methacrylyloxyethyl-2, 2, 4-triethylhexamethylene dicarbamate Bis-GMA: 2,2-bis[4[2-hydroxy-3-methacryloxypropoxy)phenyl] propane NPGDMA: Neopentyl glycol dimethacrylate | | | | | | | | | | |

### <Producing method of a dental restorative material composition>

A dental restorative composition material was produced by mixing a (meth)acrylate compound, an inorganic filler, and the organic-inorganic composite filler produced in advance at ratios shown in Tables 2 and 3, adding camphorquinone (CQ) as the photopolymerization initiator and dimethylaminoethyl methacrylate to the mixture, and stirring and mixing the materials.

### (1) Evaluation of flexural strength (a flexural strength test according to JIS T6514:2005 (ISO4049:2000))

The dental restorative composition material was pressed to and contacted with a metal mold being 2mmx2mmx25mm by to a glass plate through cellophane, and 9 positions of the dental restorative composition material were irradiated from the upper side of one side for 10 seconds per position by a visible ray irradiator (commercial name: G-LIGHT, produced by GC Corporation) (this irradiation was performed 18 times in total to the front surface and rear surface). The irradiated sample was dipped in water for 24 hours, and subjected to a three-point flexural test by a universal testing machine (commercial name: AUTOGRAPH, produced by Shimadzu Corporation) at a span length of 20mm and a cross head speed of 1 mm/min. The strength of the sample was evaluated based on the following reference. These results were shown in Table 3 collectively.
Excellent: 150MPa or more
Good: 100MPa or more and less than 150MPa
Poor: Less than 100MPa

### (2) Evaluation of uneven abrasion

The dental restorative material composition was pressed to and contacted with a metal mold having an inner diameter of 20mm and a thickness of 2mm by a glass plate through cellophane. The pressed composition was irradiated from the upper side of one side for 180 seconds by a visible ray irradiator (commercial name: LABOLIGHT LV-II, produced by GC Corporation) so that the entire composition was irradiated. The surface of the sample was polished by waterproof polishing paper of #1000, polishing paper of #1500, and a polishing tool (commercial name: POLISHING FELT DIAPOLISHER PASTE, produced by GC Corporation) in this order, so that a testing body was made. The testing body was subjected to brush abrasion 50,000 times (reciprocally) at a load of 500g in a solution, which has a ratio of a toothpaste (commercial name: White & White, produced by Lion Corporation) and distilled water being 1 : 2, by using a toothbrush abrasion testing machine. After the toothbrush abrasion test, the surface roughness on the abraded surface (Ra value) was measured by a surface roughness testing machine, and the uneven abrasion was evaluated based on a following reference. These results are shown in Tables 2 and 3 collectively.
Excellent: Less than 0.15µm
Good: 0.15µm or more and less than 0.30µm
Poor: 0.30µm or more

### (3) Evaluation of polishing property

The dental restorative material composition was pressed to and contacted with a metal mold having an inner diameter of 20mm and a thickness of 2mm by a glass plate through cellophane. The pressed composition was irradiated from the upper side of one side for 180 seconds by a visible ray irradiator (commercial name: LABOLIGHT LV-II, produced by GC Corporation) so that the entire composition was irradiated. The sample surface was subjected to softly polishing by a waterproof polishing paper of #600 so that a testing body was made. The testing body was polished for 1 minute by a polishing material (commercial name: DIA SHINE, produced by GC Corporation). Then, the glossiness on the polished surface was measured by a glossmeter (commercial name: VG-2000, produced by Nippon Denshoku Industries Co., Ltd.), and polishing property was evaluated by the following reference. These results are shown in Tables 2 and 3 collectively.
Excellent: 60% or more
Good: 50% or more and less than 60%
Poor: Less than 50%

### (4) Evaluation of lubrication

The dental restorative material composition was pressed to and contacted with a metal mold having an inner diameter of 20mm and a thickness of 2mm by a glass plate through cellophane. The pressed composition was irradiated from the upper side of one side for 180 seconds by a visible ray irradiator (commercial name: LABOLIGHT LV-II, produced by GC Corporation) so that the entire composition was irradiated. The sample surface was subjected to softly polishing by a waterproof polishing paper of #600, and was further polished for 1 minute by a polishing tool (commercial name: POLISHING FELT, produced by GC Corporation) adhered with a polishing material (commercial name: DIA POLISHER PASTE, produced by GC Corporation). Then, a surface roughness (Ra value) was measured by a surface roughness testing machine, and the lubrication was evaluated based on the following reference. These results are shown in Tables 2 and 3 collectively.
Excellent: Less than 0.1µm
Good: 0.1µm or more and less than 0.2µm
Poor: 0.2µm or more

**Table 2 (% by weight)**

| | | Example 1 | Example 2 | Example 3 | Example 4 | Example 5 | Example 6 | Example 7 | Example 8 | Example 9 |
|---|---|---|---|---|---|---|---|---|---|---|
| A | A1 | 40 | 34 | | | | | | | |
| | A2 | | | 40 | 16 | 50 | | | | |
| | A3 | | | | | | 26 | | | |
| | A4 | | | | | | | 40 | | |
| | A5 | | | | | | | | 50 | |
| | A6 | | | | | | | | | 20 |
| Total of A | components | 40 | 34 | 40 | 16 | 50 | 26 | 40 | 50 | 20 |
| B | UDMA | | 20 | 20 | 18 | | 18 | 16 | | 30 |
| | Bis-GMA | 16 | | | | 16 | | | 8 | |
| | NPGDMA | | | | 6 | | | | 6 | |
| | Particle Diameter 0.1µm | | | 39.7 | | | | 13 | | |
| | Particle Diameter 0.4µm | 43.7 | 45.7 | | 59.7 | 33.7 | 55.7 | 30.7 | 35.7 | 49.7 |
| D | CQ | 0.1 | 0.1 | 0.1 | 0.1 | 0.1 | 0.1 | 0.1 | 0.1 | 0.1 |
| | DMA | 0.2 | 0.2 | 0.2 | 0.2 | 0.2 | 0.2 | 0.2 | 0.2 | 0.2 |
| Ratio of B compnents in A conponents | | 28 | 28 | 29 | 29 | 29 | 19 | 29 | 28 | 40 |
| Ratio of B compnents in composition | | 27.2 | 29.5 | 31.6 | 28.6 | 30.5 | 22.9 | 27.6 | 28 | 38 |
| Difference between ratios | | 0.8 | 1.5 | 2.6 | 0.4 | 1.5 | 3.9 | 1.4 | 0 | 2 |
| Strength | | Excellent | Excellent | Excellent | Excellent | Excellent | Excellent | Excellent | Good | Excellent |
| Uneven abrasion | | Excellent | Excellent | Excellent | Excellent | Excellent | Excellent | Excellent | Excellent | Excellent |
| Polishing property | | Excellent | Excellent | Excellent | Excellent | Excellent | Excellent | Excellent | Excellent | Excellent |
| Lubrication | | Excellent | Excellent | Excellent | Excellent | Excellent | Excellent | Excellent | Excellent | Excellent |

**Table 3 (% by weight)**

| | | Comparative example 1 | Comparative example 2 | Comparative example 3 | Comparative example 4 | Comparative example 5 | Comparative example 6 | Comparative example 7 | Comparative example 8 |
|---|---|---|---|---|---|---|---|---|---|
| A | A1 | | | | | 50 | 20 | | |
| | A2 | | | | 55 | | | | 30 |
| | Acomp.1 | | | 8 | | | | 40 | |
| | Acomp.3 | 40 | | | | | 20 | | |
| | Acomp.2 | | 40 | | | | | | |
| Total of A components | | 40 | 40 | 8 | 55 | 50 | 40 | 40 | 30 |
| B | UDMA | 10 | 10 | 25 | 12 | 25 | | 10 | 12 |
| | Bis-GMA | | | | | | 10 | | |
| | NPGDMA | 6 | 6 | 12 | | | 6 | 6 | |
| C | Particle Diameter 0.1µm | | | | | | | | 57.7 |
| | Particle Diameter 0.4µm | 43.7 | 43.7 | 54.7 | 32.7 | 24.7 | 43.7 | 43.7 | |
| D | CQ | 0.1 | 0.1 | 0.1 | 0.1 | 0.1 | 0.1 | 0.1 | 0.1 |
| | DMA | 0.2 | 0.2 | 0.2 | 0.2 | 0.2 | 0.2 | 0.2 | 0.2 |
| Ratio of B compnents in A conponents | | 50 | 50 | 28 | 28 | 28 | 11 | 10 | 29 |
| Ratio of B compnents in composition | | 36 | 36 | 39.2 | 27.4 | 39 | 20.4 | 20 | 20.7 |
| Difference between ratios | | 14 | 14 | 11.2 | 0.6 | 11 | 9.4 | 10 | 8.3 |
| Strength | | Good | GooGood | Good | Poor | Good | Excellent | Excellent | Excellent |
| Uneven abrasion | | Poor | Poor | Poor | Excellent | Poor | Good | Poor | Poor |
| Polishing property | | Excellent | Excellent | Excellent | Excellent | Excellent | Good | Excellent | Excellent |
| Lubrication | | Excellent | Excellent | Excellent | Excellent | Excellent | Good | Excellent | Excellent |

## Claims

1. A dental restorative material composition comprising:
10 to 50% by weight of an organic-inorganic composite filler having an average particle diameter of 5 to 60µm, which is produced by mixing a (meth) acrylate compound and 60 to 90% by weight of an inorganic filler having an average primary particle diameter of 0.1 to 0.4µm curing the mixture, and pulverizing the cured material;
a (meth)acrylate compound;
an inorganic filler having an average primary particle diameter of 0.1 to 0.4µm; and
a photopolymerization initiator,
wherein a difference between a ratio (% by weight) of the (meth)acrylate compound in the organic-inorganic composite filler and a ratio (% by weight) of the (meth)acrylate compound in the entire composition is within ±5 (% by weight).
